(19) Europäisches Patentamt
European Patent Office
Office européen des brevets

(11) Publication number: 0 113 709 B1

(12) EUROPEAN PATENT SPECIFICATION

(45) Date of publication of patent specification: 08.10.86

(51) Int. Cl.$^4$: C 07 C 27/06, C 07 C 31/04, C 07 C 69/06 // B01J23/86

(21) Application number: 82901987.6

(22) Date of filing: 06.07.82

(86) International application number: PCT/NO82/00040

(87) International publication number: WO 84/00360 02.02.84 Gazette 84/03

# (54) METHOD IN THE PRODUCTION OF METHYL FORMATE AND METHANOL IN A LIQUID PHASE.

(43) Date of publication of application: 25.07.84 Bulletin 84/30

(45) Publication of the grant of the patent: 08.10.86 Bulletin 86/41

(84) Designated Contracting States: AT BE CH DE FR GB LI LU NL SE

(56) References cited:
DE-B-2 531 131
DE-C- 809 803
DE-C- 860 048
DE-C- 902 375
GB-A- 668 342
NO-C- 30 784
SE-C- 138 565

Journal of the American Chemical Society, 101:24, 1979, J.S. Bradley, "Homogeneous Carbon Monoxide Hydrogenation to Methanol Catalyzed by Soluble Ruthenium Complexes", pagea 7419-7421.

(73) Proprietor: SINTEF
Gloshaugen
N-7034 Trondheim NTH (NO)

(72) Inventor: ONSAGER, Olav, T.
Arkitekt Christies gate 8
N-7000 Trondheim (NO)

(74) Representative: Goldin, Douglas Michael et al
J.A. KEMP & CO. 14, South Square Gray's Inn
London WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 113 709 B1

**Description**

The present invention relates to a novel catalyst and to its use in a process for the production of methyl formate and methanol from carbon monoxide and hydrogen — so-called syngas.

The production of methyl formate and methanol is carried out in one step and is catalysed by a system of catalysts comprising an alkali or alkali-earth metal alcoholate and a heterogenous Cu-Cr oxide catalyst (as hereinafter defined). The reaction occurs in a liquid phase.

Methanol and methyl formate are formed by a chemical reaction of carbon monoxide and hydrogen according to the following total reactions:

| | | | |
|---|---|---|---|
| I. | CO carbon monoxide | $+ 2 H_2 \rightarrow$ hydrogen | $CH_3OH$ methanol |
| II. | 2 CO carbon monoxide | $+ 2 H_2 \rightarrow$ hydrogen | $HCOOCH_3$ methyl formate |

It is previously known to provide methyl formate from methanol and carbon monoxide in the presence of an alkali or earth alkali metal alcoholate (cf. DE—AS 1 147 214, Norw. Specification 135 749), and it is, furthermore, known that methanol can be produced by hydrogenolysis of methyl formate. DE—PS 902 375, thus, indicates a method, wherein methyl formate and hydrogen are reacted in a catalytic gas phase reaction for form methanol.

It is, furthermore, known to manufacture methyl formate and methanol in a catalytic gas phase reaction at elevated temperatures, although this has not been utilized for industrial production at a large scale.

Also, it has been suggested to carry out the reactions I and II in one and the same step (cf. DE—PS 809 803), where the formation of methanol is indicated. This method has not been utilized industrially so far, since the catalyst systems stated have low productivity and are rapidly deactivated. Industrial production of methanol today is carried out by a catalytic gas phase reaction of carbon monoxide and hydrogen by the aid of a heterogeneous metal catalyst. The reaction takes place at a pressure of approx. 100 atm. and a temperature of approx. 250°C. The gas phase reaction has the disadvantage that relatively large amounts of gas must be recompressed and recirculated to the reactor, the conversion in each passage through the reactor is relatively low. This disadvantage is strongly enhanced by the fact that the cost of energy is rapidly increasing.

EP—A — 13,005 describes a process for the production of methanol, ethylene glycol and ethanol, or carboxylate derivatives thereof, directly from hydrogen and carbon monoxide, but the catalyst comprises a solubilized ruthenium carbonyl complex dissolved in a solvent which has a dielectric constant of at least two.

GB 668,342 describes a process for the production of methanol from hydrogen and carbon monoxide via a methyl formate intermediate, using a copper or chromium catalyst, particularly copper chromite.

The present invention provides a copper-chrome oxide catalyst characterised in that it is obtained by the thermal decomposition of

$$Cu(OH)(NH_4)CrO_4$$

in an inert atmosphere at a temperature below 320°C and reduced with hydrogen in accordance with known procedures and a method for the production of methyl formate and/or methanol comprising reacting a gas comprising carbon monoxide and hydrogen in the presence of a catalyst system, comprising a metal alcoholate and a copper-chrome oxide, wherein the methanol and methyl formate are coproduced in a liquid phase in one reaction step characterised in that the copper-chrome oxide catalyst is obtained by thermal decomposition of

$$Cu(OH)(NH_4)CrO_4$$

in an inert atmosphere at a temperature below 320°C and subsequently reduced with hydrogen in accordance with known procedures.

In connection with a preferred aspect of the present invention it was surprisingly found that the product methyl formate together with methanol are advantageously removed from the reaction zone as a gas simultaneously with their formation by the chemical reaction in the liquid phase. This fact indicates that methanol is not formed *via* methyl formate as an intermediate product as conventionally assumed, but rather by a reaction which is independent of the methyl formate concentration, even though the mechanism of such reaction is not known.

The principle of driving-off produced methanol/methyl formate from the reactor brings several advantages, technically speaking, which are so important that they will make the process technically/economically competitive.

The chemical reactions taking place at the formation of methyl formate and methanol involves the liberation of considerable quantities of heat. Since the process i.a. requires defined temperatures in the reactor, heat must be removed from the zone of reaction. The reaction occurring in a liquid phase, this can be done in a simple and efficient manner as compared to a gas phase reactor. As the product, furthermore, is driven off from the liquid phase and removed from the reactor together with non-reacted carbon monoxide and hydrogen, the reaction heat will gradually be compensated by loss of heat due to the evaporation of reaction products. As regards heat economy, this is very advantageous, since the quantities of energy that would otherwise be necessary in the reactor for cooling, can thus be reduced. The evaporation of the product also in an advantageous manner renders it possible to achieve an indirect heat control of the process by varying the amount of driven off product, since the temperature of the reactor can be adjusted in this manner.

By removing the product as a gas process technical advantages associated with the catalyst, its activity and lifetime and and its handling in the process are also achieved. The catalyst, which is present as suspended material and dissolved salt (alcoholate) in the reaction medium, will not be removed, but on the contrary remain in the reactor when the product is driven off. This means the elimination of any necessary separation to remove catalyst from product in case the product had been removed from the reactor in the liquid phase. The catalyst can, thus, be handled *via* a separate process flow for any desired supply of fresh or regeneration of used catalyst.

In connection with the present invention it was also surprisingly found that there is a connection between the lifetime of the catalyst and the utilization of the principle of driving off the products from the reaction medium. The principle of driving off methyl formate from the reaction medium can, thus, suitably be utilized to minimize the concentration of methyl formate in order to increase the lifetime of the catalyst.

The reaction according to the invention occurs in a liquid reaction phase in the presence of an alkali or earth alkali metal alcholoate, preferably an alcoholate formed from methanol, particularly barium methoxide, and the subject heterogeneous catalyst suspended in the liquid reaction mixture. The pressure and the temperature of the reaction may be varied within wide limits. Preferably, pressures from 10 to 100 bar are used. The temperature is chosed to achieve a practical rate of the reaction. Preferably, the reaction is carried out at a temperature in the range of 50°C to 240°C.

It is known to produce "copper chromite" catalysts by calcination of a chemical compound with an approximate composition $Cu(OH)(NH_4)CrO_4$ in the presence of air at a temperature of approximately 350°C and subsequent reduction of the product with hydrogen. According to the present invention a specific Cu-Cr oxide catalyst is used, which is produced when the above mentioned calcination process in air is instead carried out in an inert atmosphere, e.g. argon, helium, nitrogen or similar inert gases, and is subsequently reduced with hydrogen according to known methods. It is not known in detail what chemical changes occur to the catalyst during thermal decomposition in an inert atmospere as compared to the known method of calcination. The thermal decomposition of $Cu(OH)(NH_4)CrO_4$ in an inert atmosphere can be carried out at a temperature in the range of 250—500°C, most suitably in the range of 270—320°C.

In addition to the above mentioned Cu-Cr oxide component, the catalyst system can naturally also comprise other components or carriers conventionally utilized in connection with heterogeneous hydrogenation catalysts, as for instance ZnO, $Al_2O_3$, $Fe_2O_3$, MnO, MgO, CaO, BaO, $SiO_2$, activated carbon and the like.

The reaction occurs in a technical reactor that is known for solid/liquid phase/gas systems. Preferably a kind of reactor is chosen that is characterized by good mixture of gas/liquid/solids. In the reaction methyl formate as well as methanol are formed, and the ratio between said components in the product can be varied within wide limits by changing the process conditions and the composition of the catalyst.

Methanol naturally functions as a solvent for the reaction. Other organic solvents, as for instance higher alcohols, ether, saturated hydrocarbons and aromatics could also be utilized. A syngas free of water, sulphur and $CO_2$, and wherein the molar ratio of carbon monoxide and hydrogen can be varied, is used in the present method.

On account of the conversion rate as well as the selectivity regarding the conversion to the desired products it is critical that the reaction takes place in an approximately anhydrous reaction medium.

Methyl formate and methanol are recovered as top and bottom products respectively in a separation column. Part of the produced amount of methanol is suitably returned to the reactor. The produced methanol has an especially high degree of purity (approximately anhydrous) and is, thus, especially well suited for this object. A sketch of such an embodiment of the process is shown in Figure 2.

The present invention represents an advantageous and very flexible process alternative for coproduction of methyl formate and methanol. The product composition may vary from pure methyl formate to pure methanol, and it is possible to control production according to the actual state of the market at any time. The prospects of the market of methanol as well as methyl formate are very promising, as will be known. The characteristics of methanol are studied with a view to future utilization of methanol as an energy carrier. The expected and considerable increase in production because of this will involve the need of additional plants with larger throughput. Thus, there should be a demand as well as good chances of adapting novel and improved process technology for methanol production in an industrial scale.

Methyl formate seems to become an essential intermediate in $C_1$-based chemistry, since it is reasonably priced for utilization as a raw material for a series of essential petrochemical products.

The following examples will further illuminate the object of the present invention.

Example 1

Small portions of $Cu(OH)NH_4CrO_4$ (approx. 2 g) were termally decomposed in an inert atmosphere (He). The sample was kept at maximum decomposition temperature as stated in Figure 10 for one hour. Simultaneously 120 ml/min of

helium were passed through the decomposition zone. 0,75 g of the decomposed product in 12 g methanol were then prereduced with hydrogen in a micro autoclave having a volume of 30 ml and provided with magnetic stirring and temperature control at 185°C and 100 bar hydrogen. After 16 hours the mixture was cooled to 20°C and the catalyst was separated from the methanol by centrifugation. The catalyst remained in the autoclave whereas the methanol was removed. The autoclave was then supplied with 10 g dried methanol containing 1 mole % Na methylate as catalyst 1. The conversion of carbon monoxide and hydrogen was then carried out at 130°C and 75 bar for 2,5 hours. After the test the reaction mixture was cooled to 20°C and analysed by gas/liquid chromatography (glc). The formation of methanol and methyl formate (indicated as % of weight increase) as a function of maximum decomposition temperature is shown by graph I in Figure 1. The selectivity of the reaction of CO and $H_2$ to methanol/methyl formate was more than 95% in all these tests.

For comparison $Cu(OH)NH_4CrO_4$ was calcinated in air according to the known methods and tested as a catalyst for the reaction of carbon monoxide and hydrogen according to the same method as stated above. The results are shown by graph II in Figure 1.

The results show that a catalyst that is more active in an substantial degree is provided by thermal decomposition of $Cu(OH)NH_4CrO_4$ in an inert atmosphere, as compared to the activity achieved by known processes of calcination.

## Claims

1. A method for the production of methyl formate and/or methanol comprising reacting a gas comprising carbon monoxide and hydrogen in the presence of a catalyst system, comprising an alkali or alkali-earth metal alcoholate and a copper-chrome oxide, wherein the methanol and methyl formate are coproduced in a liquid phase in one reaction step characterised in that the copper-chrome oxide catalyst is obtained by thermal decomposition of $Cu(OH)(NH_4)CrO_4$ in an inert atmosphere at a temperature below 320°C and subsequently reduced with hydrogen in accordance with known procedures.

2. A method according to claim 1 characterised in that the methyl formate and methanol are separated by fractional distillation and all of part of the methanol is recirculated to the reaction zone.

3. A method according to claim 1 or 2 characterised in that the metal alcoholate is barium methoxide.

4. A method according to any one of the preceding claims characterised in that the reaction is conducted at a temperature below 240°C and at a pressure below 100 bar.

5. A method according to any one of the preceding claims wherein the methanol and methyl formate are removed in a gaseous phase from the reaction zone.

6. A copper-chrome oxide catalyst characterised in that it is obtained by the thermal decomposition of $Cu(OH)(NH_4)CrO_4$ in an inert atmosphere at a temperature below 320°C and reduced with hydrogen in accordance with known procedures.

## Patentansprüche

1. Herstellungsverfahren für Methylformiat und Methanol, das die Reaktion eines Kohlenstoffmonoxyd und Wasserstoff aufweisenden Gases bei Anwesenheit eines Katalysatorsystems aus einem Alkali- oder Erdalkali-Alkoholat und Kupfer-Chromoxid umfaßt, wobei das Methanol und Methylformiat in flüssiger Phase in einem Reaktionsschritt zusammen hergestellt werden, dadurch gekennzeichnet, daß der Kupfer-Chromoxidkatalysator durch Wärmezersetzung von $Cu(OH)(NH_4)CrO_4$ in einer inerten Atmosphäre bei einer Temperatur unter 320 °C erhalten und danach mit Wasserstoff gemäß bekannten Verfahren reduziert wird.

2. Herstellungsverfahren nach Anspruch 1, dadurch gekennzeichnet, daß das Methylformiat und Methanol durch fraktionierte Destillation getrennt werden und das Methanol insgesamt oder teilweise in die Reaktionszone zurückgeführt wird.

3. Herstellungsverfahren nach einem der Ansprüche 1 oder 2, dadurch gekennzeichnet, daß das Metallalkoholat Bariummethoxid ist.

4. Herstellungsverfahren nach einem der vorangehenden Ansprüche, dadurch gekennzeichnet, daß die Reaktion bei einer Temperatur unter 240 °C und bei einem Druck unter 100 bar durchgeführt wird.

5. Herstellungsverfahren nach einem der vorangehenden Ansprüche, dadurch gekennzeichnet, daß das Methanol und Methylformiat in einer gasförmigen Phase aus der Reaktionszone entfernt werden.

6. Kupfer-Chromoxidkatalysator, dadurch gekennzeichnet, daß dieser durch Wärmezerlegung von $Cu(OH)(NH_4)CrO_4$ in einer inerten Atmosphäre bei einer Temperatur unter 320 °C erhalten und mit Wasserstoff nach bekannten Verfahren reduziert wird.

## Revendications

1. Procédé de production du formiate de méthyle et/ou du méthanol, comprenant l'étape consistant à faire réagir un gaz comprenant de l'oxyde de carbone et de l'hydrogène en présence d'un système de catalyseur, comprenant un alcoolate de métal alcalin ou alcalinoterreux et un oxyde de cuivre-chrome, dans lequel le méthanol et le formiate de méthyle sont co-produits dans une phase liquide en une étape réactionnelle, caractérisé par le fait que le catalyseur d'oxyde de cuivre-chrome est obtenu par décomposition thermique de $Cu(OH)(NH_4)CrO_4$ en atmosphère inerte à une température inférieur à 320°C, puis réduit par l'hydrogène conformément à des techniques connues.

2. Procédé selon la revendication 1, caractérisé

par le fait que le formiate de méthyle et le méthanol sont séparés par distillation fractionnée et en ce que tout ou partie du méthanol est recyclé dans la zone réactionnelle.

3. Procédé selon la revendication 1 ou 2, caractérisé par le fait que l'alcoolate métallique est du méthylate de baryum.

4. Procédé selon l'une quelconque des revendications précédentes, caractérisé par le fait que la réaction est conduite à une température inférieure à 240°C et sous une pression inférieure à 100 bars.

5. Procédé selon l'une quelconque des revendications précédentes, caractérisé par le fait que le méthanol et le formiate de méthyle sont éliminés en phase gazeuse de la zone réactionnelle.

6. Catalyseur d'oxyde de cuivre-chrome, caractérisé par le fait qu'il est obtenu par décomposition thermique de $Cu(OH)(NH_4)CrO_4$ en atmosphère inerte, à une température inférieure à 320°C et réduit par l'hydrogène conformément à des techniques connues.

Figure 1

Production of methanol and methyl formate from $CO/H_2$ as a function of maximum decomposition temperature for Cu(OH/$NH_4CrO_4$

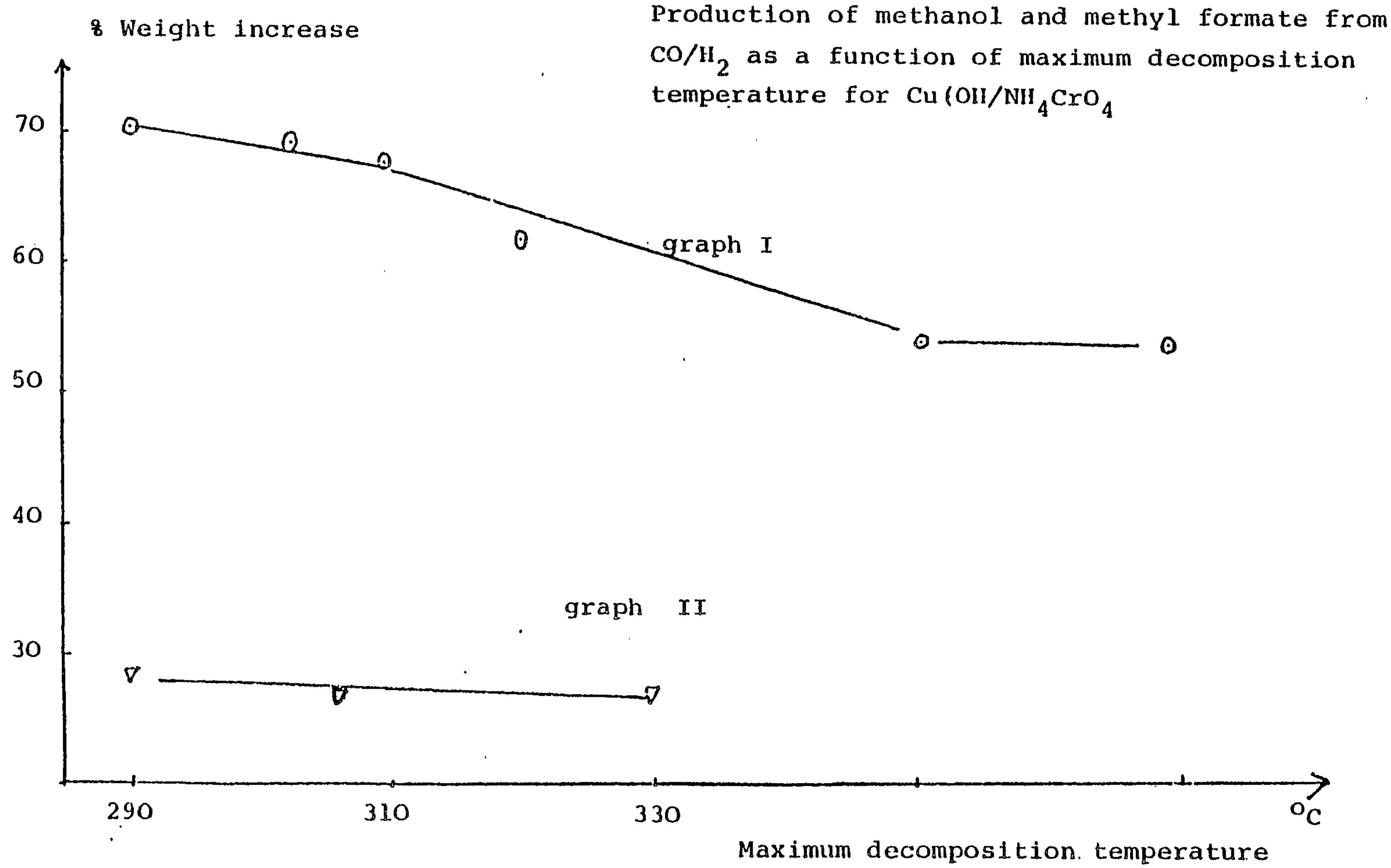

Figure 2:

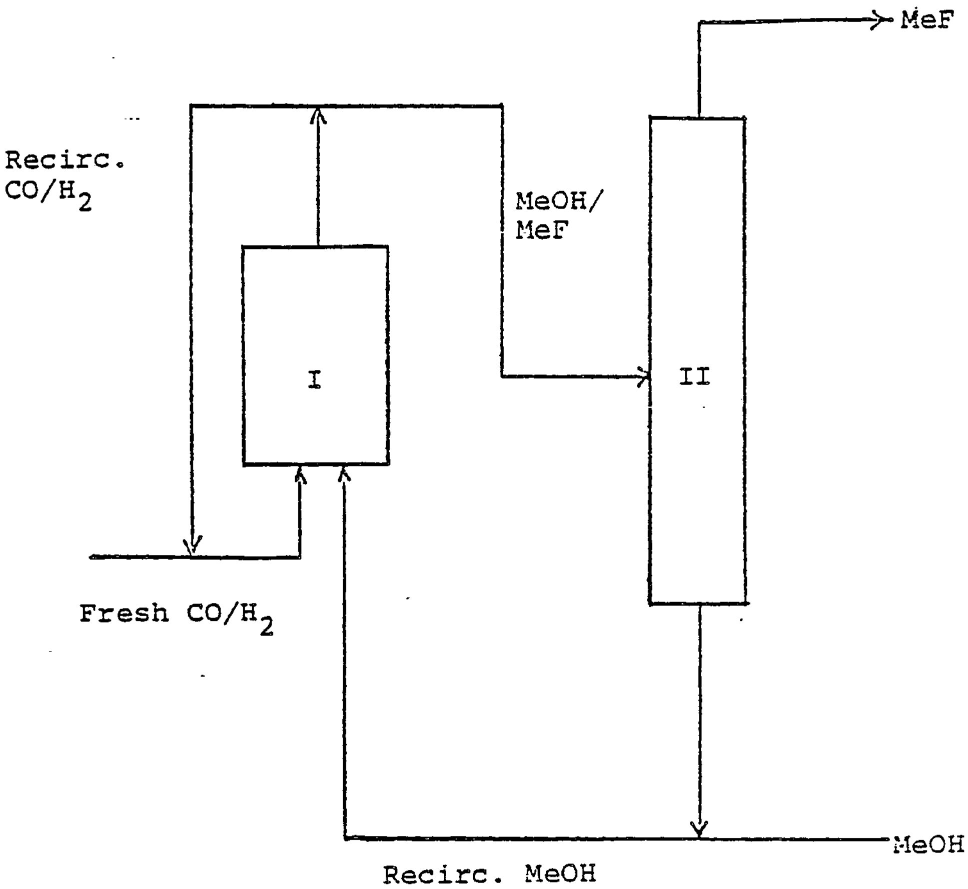


I) Reactor

II) Distillation unit